(19)

**Europäisches Patentamt**
**European Patent Office**
**Office européen des brevets**

(11) **EP 4 732 948 A1**

(12) **EUROPEAN PATENT APPLICATION**

(43) Date of publication:
29.04.2026 Bulletin 2026/18

(21) Application number: 24209261.7

(22) Date of filing: 28.10.2024

(51) International Patent Classification (IPC):
*B01L 3/00* (2006.01)  *C12N 15/10* (2006.01)
*C12Q 1/68* (2018.01)  *G01N 1/40* (2006.01)

(52) Cooperative Patent Classification (CPC):
(C-Sets available)
**B01L 3/502753; C12N 15/1006; G01N 1/40;**
B01L 2200/0631; B01L 2300/0816;
B01L 2400/0487; B01L 2400/086          (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: Imec VZW
**3001 Leuven (BE)**

(72) Inventors:
• DALBEN MADEIRA CAMPOS, Camila
**3001 Heverlee (BE)**
• HOELZ, Kathrin
**3001 Leuven (BE)**

(74) Representative: **Winger**
**Mouterij 16 bus 101**
**3190 Boortmeerbeek (BE)**

(54) **MICROFLUIDIC DEVICE FOR NUCLEIC ACID PURIFICATION**

(57)    A microfluidic device for purification of nucleic acids comprising: at least one microfluidic channel, said channel having a first surface, a second surface opposite the first surface, and connecting surfaces joining the first and second surfaces; a plurality of fixed structures within said channel, said structures being attached to at least one of the first surface or second surface of the channel, collectively occupying at least 95% of the distance between the connecting surfaces, and each structure extending across at least 95% of the distance between the surface to which it is attached and the opposite surface; at least one inlet for introducing a fluid containing nucleic acids or molecular assemblies comprising nucleic acids into the channel; and at least one outlet for collecting purified nucleic acids; wherein said fixed structures comprise a surface capable of reversibly binding either nucleic acids or molecular assemblies comprising nucleic acids.

**FIG. 1**

EP 4 732 948 A1

(52) Cooperative Patent Classification (CPC): (Cont.)

C-Sets
**C12Q 1/6806, C12Q 2565/629**

## Description

**Field of the Invention**

The present invention relates to the field of nucleic acid purification, and more specifically to microfluidic devices and methods for purifying nucleic acids using fixed structures within microfluidic channels.

**Background of the Invention**

Nucleic acids are fundamental molecules in the fields of molecular and synthetic biology, biotechnology, and medical research. The purification of these nucleic acids is a crucial step in various laboratory procedures, ensuring that the samples are of sufficient purity and concentration for downstream applications like sequencing, cloning, amplification, and analysis.

One common method for nucleic acid purification involves solid-phase extraction using functionalized beads. These beads are typically coated with hydrophobic molecules, such as C18 chains, to selectively bind nucleic acids or their assemblies. While bead-based methods are widely utilized due to their effectiveness, they present certain limitations in practical applications.

A significant challenge associated with bead-based purification is the lack of integration with other workflow steps. The process often requires multiple manual handling stages, including bead addition, incubation, separation, and washing. This complexity can lead to increased processing times and a higher risk of sample loss or contamination. The necessity for manual intervention also makes automation difficult, which is a drawback for high-throughput settings where efficiency and consistency are paramount.

Additionally, the physical nature of the beads can pose operational issues. Handling loose beads necessitates careful control to prevent unintended transfer or loss. There is also the potential for beads to cause clogging in microfluidic systems or other equipment, which can disrupt the purification process and affect overall yield and purity.

These challenges underscore the limitations of current nucleic acid purification techniques, particularly in terms of workflow integration, sample handling, and scalability. Improving these aspects would help advancing molecular biology protocols and enhancing the efficiency of nucleic acid-based applications.

However, despite the existing methodologies, there remains a need for further advancements in the field to address at least some of these challenges.

**Summary of the Invention**

It is an object of embodiments of the present invention to provide devices and methods for efficient purification of nucleic acids. This objective is accomplished by a microfluidic device and method for purification of nucleic acids according to the invention.

In the first aspect, the present invention relates to a microfluidic device for purification of nucleic acids comprising:

at least one microfluidic channel, said channel having a first surface, a second surface opposite the first surface, and connecting surfaces joining the first and second surfaces;

an ensemble of one or more fixed structures within said channel, each structure being attached to at least one of the first surface or second surface of the channel, wherein the ensemble, including any spaces between individual structures, collectively extends over at least 95% of the distance between the connecting surfaces of the channel, and each structure extending across at least 95% of the distance between the surface to which it is attached and the opposite surface;

at least one inlet for introducing a fluid containing nucleic acids or molecular assemblies comprising nucleic acids into the channel; and

at least one outlet for collecting purified nucleic acids;

wherein said fixed structures comprise a surface capable of reversibly binding either nucleic acids or molecular assemblies comprising nucleic acids.

In embodiments, the density and/or arrangement of said fixed structures may be such that, when the binding surfaces are free of bound nucleic acids, passing 1 $\mu$L of a sample containing 1 ng/$\mu$L of nucleic acids or molecular assemblies comprising nucleic acids through the microfluidic channel at a flow rate of 1 $\mu$L/min results in the capture of at least 50% of the nucleic acids or molecular assemblies by the fixed structures. This allows for efficient capture of nucleic acids.

In embodiments, the fixed structures may be three-dimensional structures, and the ensemble may form an array of said three-dimensional structures. The three-dimensional structures provide a high surface area for binding.

In embodiments, the average distance between a fixed structure and its closest neighbor may be from 1 $\mu$m to 200 $\mu$m, preferably from 5 $\mu$m to 100 $\mu$m, yet more preferably from 5 $\mu$m to 50 $\mu$m. This spacing allows for optimal flow and capture.

In embodiments, the height of the three-dimensional structures within the array may range from 10 $\mu$m to 500 $\mu$m, preferably between 20 $\mu$m and 200 $\mu$m. In embodiments, the width or diameter of the three-dimensional structures within the array may range from 1 $\mu$m to 100 $\mu$m, with a preferred range of 5 $\mu$m to 50 $\mu$m. In embodiments, spacing between the three-dimensional structures within the array may range from 1 $\mu$m to 200 $\mu$m, preferably between 5 $\mu$m and 100 $\mu$m, yet more preferably from 5 to 50 $\mu$m. In embodiments, the three-dimensional structures density within the array may range from 1,000 to 1,000,000 three-dimensional structures per cm$^2$, with a preferred range of 10,000 to 250,000 three-dimensional structures per cm$^2$.

In embodiments, the fixed structures may be pillars. Pillars are easy to fabricate and provide good flow characteristics.

In embodiments, the fixed structures may be arranged in a regular configuration, preferably in a regular staggered configuration. The staggered arrangement maximizes capture efficiency.

In embodiments, the ensemble may comprise a mesh. A mesh structure provides a high surface area in a compact volume.

In embodiments, the fixed structures may comprise a porous material with pores having a pore size of from 50 to 500 nm. The porous material increases the surface area for binding.

In embodiments, the porous material may have a pore size of from 50 to 60 nm, and the device may be for purification of nucleic acids less than 35 nucleotides in length. The pore size is optimized for short nucleic acids.

In embodiments, the porous material may have a pore size of from 61 nm to 199 nm, and the device may be for purification of nucleic acids greater than 35 nucleotides and up to 80 nucleotides in length. The pore size is optimized for medium length nucleic acids.

In embodiments, the porous material may have a pore size of from 200 to 300 nm, and the device may be for purification of nucleic acids greater than 80 nucleotides in length. The pore size is optimized for long nucleic acids.

In embodiments, each of the fixed structures may be attached to both the first surface and the second surface. Attachment to both surfaces provides stability and uniform flow.

In embodiments, the one or more fixed structures may be made of the same material as a substrate comprising the first surface of the microfluidic device. Using the same material simplifies fabrication.

In embodiments, the one or more fixed structures may be made of silicon or metal, preferably silicon. Silicon is readily available and easy to process.

In embodiments, the surface capable of reversibly binding either nucleic acids or molecular assemblies comprising nucleic acids may comprise entities selected from the list consisting of hydrophobic groups such as C18 chains, nucleic acid sequences complementary to the target nucleic acids to be purified, streptavidin, and biotin. These entities enable specific and reversible capture of nucleic acids or molecular assemblies comprising nucleic acids.

In embodiments, the device may comprise at least two outlets. Multiple outlets allow for separate collection of waste and product.

In embodiments, at least one of the outlets may be fluidly connected to at least one of the inlets to recirculate fluid through the channel. Recirculation can increase capture efficiency.

In embodiments, the device may comprise at least two inlets. Multiple inlets enable introduction of different fluids for binding and elution.

In the second aspect, the present invention relates to a method of purifying nucleic acids comprising:

Introducing a fluid containing nucleic acids or molecular assemblies comprising nucleic acids into a microfluidic device according to any embodiments of the first aspect;
Allowing the nucleic acids to bind to the fixed structures;
washing away unbound material; and
eluting the bound nucleic acids to collect purified nucleic acids.

In embodiments, the washing step may include introducing a wash buffer through the channel. The wash buffer removes unbound material.

In embodiments, the microfluidic device may comprise at least two inlets, wherein the introducing step is performed into a first inlet and wherein the washing step includes introducing the wash buffer through the channel via a second inlet, different from the first inlet. Using separate inlets prevents mixing of binding and wash solutions.

In embodiments, the eluting step may include introducing a cleavage reagent through the channel, providing heat to the bound nucleic acids, changing buffer composition to alter binding stringency, or a combination thereof. The cleavage reagent, change in buffer composition, or heat disrupts binding to release the nucleic acids.

In embodiments, the fluid containing nucleic acids may be introduced into the channel under pressure-driven flow. Pressure-driven flow enables control over flow rates.

In the third aspect, the present invention relates to a process for nucleic acid synthesis, comprising:

Synthesizing nucleic acid fragments; and
Purifying the synthesized nucleic acid fragments using the method according to any embodiments of the second aspect.

In embodiments, the surface may comprise entities selected from the list consisting of hydrophobic groups such as C18 chains, streptavidin, biotin, and (complementary) nucleic acid sequences,. These entities enable capture of synthesized nucleic acid fragments.

In embodiments, the process may further comprise the following steps after the Purifying step:
Assembling the purified nucleic acid fragments into larger nucleic acid fragments, then purifying the assembled larger nucleic acid fragments using the method according to any embodiments of the second aspect, wherein the surface capable of reversibly binding either nucleic acids or molecular assemblies comprising nucleic acids comprises nucleic acid sequences complementary to the target nucleic acids to be purified, wherein completely assembled fragments are retained and incompletely assembled fragments are removed. This allows for purification of the final assembled product.

In the fourth aspect, the present invention relates to a system for synthesis and purification of nucleic acids comprising:

> a synthesis module for synthesizing nucleic acids fragments; and
> a first microfluidic device according to any embodiments of the first aspect;
> wherein the synthesis module and microfluidic device are fluidically connected.

In embodiments, the surface capable of reversibly binding either nucleic acids or molecular assemblies comprising nucleic acids may comprise entities selected from the list consisting of hydrophobic groups such as C18 chains, streptavidin, and biotin.. These entities enable capture of synthesized fragments.

In embodiments, the system may further comprise an assembling module for assembling the purified nucleic acid fragments into larger nucleic acid fragments, and a second microfluidic device according to any embodiments of the first aspect, wherein the surface capable of reversibly binding either nucleic acids or molecular assemblies comprising nucleic acids comprises nucleic acid sequences complementary to the target nucleic acids to be purified. This enables purification of the assembled product.

In embodiments, the synthesis module may comprise a channel having a first surface, a second surface opposite the first surface, and connecting surfaces joining the first and second surfaces; an ensemble of one or more fixed structures within said channel, each structure being attached to at least one of the first surface or second surface of the channel, wherein the ensemble, including any spaces between individual structures, collectively extends over at least 95% of the distance between the connecting surfaces of the channel, and each structure extending across at least 95% of the distance between the surface to which it is attached and the opposite surface. The fixed structures in the synthesis module provide a high surface area for efficient synthesis. In embodiments, each fixed structure may be structurally identical to the fixed structures within the microfluidic channel of the microfluidic deice for purification of nucleic acids.

In embodiments, the system may further comprise a control unit for automated operation of the synthesis and purification processes. Automation increases throughput and reproducibility.

In embodiments, the synthesis module may synthesize nucleic acids using either phosphoramidite chemistry or enzymatic synthesis. In the case of enzymatic synthesis, the process can be controlled through the use of enzymes such as ligases and polymerases, along with reagents, and may also use light, heat, or electrochemistry. In the case of phosphoramidite chemistry, the synthesis is typically controlled through the use of reagents, with light and heat potentially used for specific steps such as deprotection or drying. These are common methods for nucleic acid synthesis.

In the fifth aspect, the present invention relates to a computer program comprising instructions to cause the system according to any embodiments of the fourth aspect to execute the steps of the process according to any embodiments of the third aspect.

In the sixth aspect, the present invention relates to a computer-readable medium having stored thereon the computer program according to the fifth aspect.

It is an advantage of embodiments of the present invention that the use of fixed pillars allows for easier fabrication compared to bead-based purification systems.

It is an advantage of embodiments of the present invention that sample manipulation is reduced due to the integration of the purification process within a microfluidic device.

It is a further advantage of embodiments of the present invention that hands-on time is decreased, leading to increased yield in nucleic acid purification.

It is an advantage of embodiments of the present invention that the fixed pillars enable integration with other steps in the nucleic acid synthesis and purification workflow, overcoming the lack of integration associated with bead-based methods.

It is a further advantage of embodiments of the present invention that the operation is simplified, as there is no risk of beads being flushed away or causing clogging in the microfluidic channels.

It is an advantage of embodiments of the present invention that the pillars provide a well-controlled three-dimensional structure, maximizing the capture surface area in a reproducible manner.

It is a further advantage of embodiments of the present invention that the fixed structures allow for automated operation and can be incorporated into a fully automated system for nucleic acid synthesis and purification.

It is an advantage of embodiments of the present invention that the purification device can handle pressure-driven flow sufficient to capture a high number of nucleic acid molecules per unit area.

It is a further advantage of embodiments of the present invention that the pillars can be functionalized with various capture chemistries, providing flexibility and efficiency in purification methods.

It is an advantage of embodiments of the present invention that the use of pillars reduces the risk of clogging and sample loss, leading to a more efficient and reliable purification process.

It is a further advantage of embodiments of the present invention that the device design allows for higher capacity purification by arranging multiple purification modules in series or parallel configurations.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

**Brief description of the drawings**

Fig. 1 is a vertical cross-sectional view of a microfluidic device according to embodiments of the present invention.

Fig. 2 is a vertical cross-sectional view of a microfluidic device according to other embodiments of the present invention.

Fig. 3 is a vertical longitudinal cross-sectional view of the microfluidic device of Fig. 1 according to embodiments of the present invention.

Fig. 4 is a vertical longitudinal cross-sectional view of the microfluidic device of Fig. 2 according to embodiments of the present invention.

Fig. 5 is a schematic view of a system for synthesis and purification of nucleic acids according to embodiments of the present invention.

Fig. 6 is a flowchart of a method of purifying nucleic acids according to embodiments of the present invention.

Fig. 7 is a schematic view of a process for nucleic acid synthesis according to embodiments of the present invention.

Fig. 8 is a top view of a fixed structure having a surface comprising nucleic acid sequences complementary to target nucleic acids according to embodiments of the present invention.

Fig. 9 is a top view of a fixed structure having a surface comprising C18 chains according to embodiments of the present invention.

In the different figures, the same reference signs refer to the same or analogous elements.

**Detailed description of Illustrative Embodiments**

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The following terms are provided solely to aid in the understanding of the invention.

As used herein, and unless otherwise specified, the term "microfluidic device" refers to a device that has one or more channels with at least one dimension less than 1 mm. It is typically capable of handling and manipulating fluid volumes smaller than a milliliter.

As used herein, and unless otherwise specified, the term "nucleic acids" refers to biopolymers composed of nucleotide monomers, such as deoxyribonucleic acid (DNA), ribonucleic acid (RNA), or xeno(biotic) nucleic acid (XNA) regardless of their origin. It encompasses single-stranded and double-stranded forms. It also encompasses synthetic analogs as well as canonical and non-canonical structures. Typical examples are synthetic single-stranded oligonucleotides.

As used herein, and unless otherwise specified, the term "microfluidic channel" refers to a channel or passageway within a microfluidic device that has at least one dimension less than 1 mm, and through which fluids can flow. The microfluidic channel can have various cross-sectional shapes, such as rectangular, square, circular, or elliptical, and can be fabricated using various materials, such as glass, silicon, or polymers.

As used herein, and unless otherwise specified, the term "fixed structures" refers to solid features attached to at least one of the first surface or the second surface within the microfluidic channel, extending into the channel space. These structures are designed to increase surface area for interactions with fluids passing through the channel. Examples of fixed structures include pillars (e.g., etched into the substrate) and porous materials such as meshes or porous pillars, attached within the channel.

As used herein, and unless otherwise specified, the term "pillar" refers to a vertical structural element within the microfluidic channel that extends from one surface towards the opposite surface. Pillars have a height substantially greater than their width or diameter, typically with an aspect ratio (height to width) of at least 2:1, preferably at least 3:1 and typically up to 50:1. Pillars may have various cross-sectional shapes such as circular, square, rectangular, or polygonal. They are distinct from other fixed structures such as ridges or grooves in that pillars are discrete elements surrounded by fluid flow on multiple sides. In embodiments, the height of the pillars may range from 10 μm to 500 μm, preferably

between 20 μm and 200 μm. In embodiments, the width or diameter of the pillars may range from 1 μm to 100 μm, with a preferred range of 5 μm to 50 μm. In embodiments, spacing between pillars may range from 1 μm to 200 μm, preferably between 5 μm and 100 μm, yet more preferably from 5 to 50 μm. In embodiments, the pillar density may range from 1,000 to 1,000,000 pillars per cm$^2$, with a preferred range of 10,000 to 250,000 pillars per cm$^2$.

As used herein, and unless otherwise specified, the phrase "within said channel" means that the fixed structures are located inside the microfluidic channel, between the first and second surfaces and within the boundaries defined by the connecting surfaces.

As used herein, and unless otherwise specified, the phrase "attached to at least one of the first surface or second surface of the channel" means that each fixed structure is physically connected to either the first surface, the second surface, or both, of the microfluidic channel, providing structural stability and proper positioning within the channel.

As used herein, and unless otherwise specified, the phrase "the ensemble, including any spaces between individual structures, collectively extends over at least 95% of the distance between the connecting surfaces of the channel" means that the combined width of the ensemble of fixed structures and the gaps or spaces between them spans at least 95% of the total width of the microfluidic channel measured between the side walls (connecting surfaces).

As used herein, and unless otherwise specified, the phrase "each structure extending across at least 95% of the distance between the surface to which it is attached and the opposite surface" means that for each fixed structure, its dimension perpendicular to the surface to which it is attached extends at least 95% of the distance toward the opposing surface, leaving at most 5% gap between the top of the structure and the opposing surface.

As used herein, and unless otherwise specified, the term "molecular assemblies comprising nucleic acids" refers to any molecule that includes one or more nucleic acid sequences.

As used herein, and unless otherwise specified, the term "surface capable of reversibly binding either nucleic acids or molecular assemblies comprising nucleic acids" refers to a surface that has chemical or physical properties that allow nucleic acids or molecular assemblies containing nucleic acids to bind under certain conditions and to be released under different conditions without permanently altering the nucleic acids; examples include surfaces modified with specific functional groups or affinity ligands.

As used herein, and unless otherwise specified, the term "reversibly binding" means that the binding interaction between the nucleic acids (or molecular assemblies comprising nucleic acids) and the surface can be reversed by changing conditions such as pH, ionic strength, temperature, introducing a cleavage reagent, or applying other elution methods.

As used herein, and unless otherwise specified, the phrase "porous material with pores having a pore size of from 50 to 500 nm" refers to a solid material containing pores (e.g., channels or interconnected voids) wherein at least one of said pores has at least one transversal cross-section having an equivalent diameters in the range of from 50 to 500 nanometers. Preferably, at least 50% of the pores have at least one transversal cross-section having an equivalent diameter in the range of from 50 to 500 nanometers. Preferably, at least 50% of the pores have all their transversal cross-section having an equivalent diameter in the range of from 50 to 500 nanometers. Preferably, at least 90% of the pores have all their transversal cross-section having an equivalent diameter in the range of from 50 to 500 nanometers. The equivalent diameter( De) of a pore's transversal cross-section is defined as the diameter of a circle with an equal sectional area as the sectional area (A) of the pore in the transversal cross-section.

$$D_e = \sqrt{\frac{4A}{\pi}} \text{ ,}$$

It can be measured by transmission electron microscopy. Examples include nanoporous silicon, anodic aluminum oxide membranes, or porous polymer monoliths. Such materials provide increased surface area for binding interactions and can facilitate size-based separation of nucleic acids.

The pore size affects the accessibility and binding capacity of the material for nucleic acids of different sizes.

As used herein, and unless otherwise specified, the term "mesh" refers to a structure composed of a network of interwoven or interconnected elements, which can be made of various materials, such as polymers, semiconductor (e.g. Si), metals, or ceramics. The mesh can have various pore sizes and densities, depending on the intended application. As used herein, and unless otherwise specified, the term "synthesis module" refers to a component or unit within the system configured to synthesize nucleic acid fragments, utilizing methods such as phosphoramidite-based chemical synthesis or enzymatic synthesis, and may include reaction chambers, fluidic pathways, and delivery systems for reagents.

As used herein, and unless otherwise specified, the term "assembling module" refers to a component or unit within the system designed to assemble smaller nucleic acid fragments into larger nucleic acid constructs, using techniques such as ligation, polymerase extension, or recombination, and may include reaction chambers and requisite reagents.

As used herein, and unless otherwise specified, the term "control unit" refers to an electronic device or system that manages and automates the operation of the synthesis and purification processes, including controlling fluid flow, reagent delivery, temperature, timing, and data collection, and may comprise a computer or microcontroller executing software instructions.

As used herein, and unless otherwise specified, the phrase "nucleic acid sequences complementary to the target nucleic acids to be purified" refers to sequences that are designed to hybridize specifically to the target nucleic acids through base pairing interactions, enabling selective binding and capture of the target sequences while unbound or non-complementary sequences are washed away.

As used herein, and unless otherwise specified, the term "hydrophobic groups" refers to chemical moieties attached to the surface of the fixed structures within the microfluidic device. These groups typically interact with hydrophobic moieties that may be present on molecular assemblies comprising nucleic acids. Such hydrophobic moieties on the nucleic acids may include:

Protecting groups used in nucleic acid synthesis, such as dimethoxytrityl (DMT) or other trityl groups at the 5'-end of oligonucleotides.

Hydrophobic labels or tags intentionally added to the nucleic acids, such as fluorescent dyes, biotin, or other small hydrophobic molecules.

Lipid conjugates attached to nucleic acids, as in the case of some therapeutic oligonucleotides or siRNA delivery systems.

Hydrophobic peptides or polymers conjugated to nucleic acids for various research or therapeutic applications.

The hydrophobic groups on the surface (9) may be selected from a wide range of structures, including but not limited to linear alkyl chains of various lengths (such as octyl (C8), decyl (C10), dodecyl (C12), tetradecyl (C14), hexadecyl (C16), octadecyl (C18), and eicosyl (C20)), branched alkyl chains (e.g., 2-ethylhexyl, isodecyl, isotridecyl), cyclic and polycyclic aliphatic groups (e.g., cyclohexyl, adamantyl), aromatic groups (e.g., phenyl, naphthyl, biphenyl), fluorinated alkyl chains (e.g., trifluoroethyl, perfluorooctyl), and mixed aliphatic-aromatic groups (e.g., benzyl, phenethyl). Additionally, polymeric hydrophobic groups such as polystyrene, polyethylene, and polypropylene may be employed. The term also includes commercially available chromatographic ligands like Sepharose™ (cross-linked agarose beads), Phenyl Sepharose™, Butyl Sepharose™, and Octyl Sepharose™. Silica-based hydrophobic groups such as trimethylsilyl (TMS) and dimethyloctylsilyl, as well as lipid-based groups like cholesteryl and phospholipid derivatives, are also encompassed within this definition. These hydrophobic groups can be incorporated onto the surface of the fixed structures within the microfluidic device through various chemical modification techniques, including silanization, polymer grafting, or direct chemical synthesis. The choice of hydrophobic group can be tailored to optimize the binding and purification of specific nucleic acids or molecular assemblies comprising nucleic acids, allowing for versatility in the application of the microfluidic device to various purification tasks.

As used herein, and unless otherwise specified, the term 'phosphoramidite chemistry' refers to a method of synthesizing nucleic acids using phosphoramidite nucleoside monomers. This is typically performed in a solid-phase synthesis process. The term 'enzymatic synthesis' refers to the synthesis of nucleic acids using enzymes such as polymerases or ligases, with the process being regulated by the addition of specific reagents. It may also involve the application of light and/or heat to control various steps. It may also involve electrochemical methods to control various steps.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

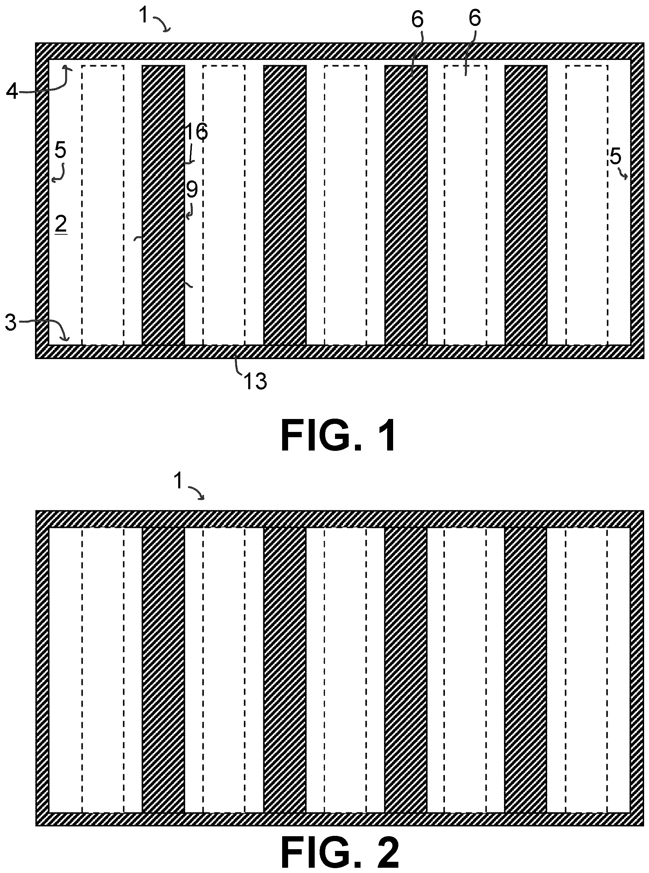

We now refer to Figures 1 to 4.

The present invention relates to a microfluidic device for purification of nucleic acids comprising: at least one microfluidic channel (2), said channel having a first surface (3), a second surface (4) opposite the first surface, and connecting surfaces (5) joining the first and second surfaces; a plurality of fixed structures (6) within said channel, said structures being attached to at least one of the first surface or second surface of the channel, collectively occupying at least 95% of the distance between the connecting surfaces, and each structure extending across at least 95% of the distance between the surface to which it is attached and the opposite surface; at least one inlet (7) for introducing a fluid containing nucleic acids or molecular assemblies comprising nucleic acids into the channel; and at least one outlet (8) for collecting purified nucleic acids; wherein said fixed structures comprise a surface (9) capable of reversibly binding either nucleic acids or molecular assemblies comprising nucleic acids.

Referring to Figure 1, it illustrates a vertical cross-sectional view of a microfluidic device (1) designed for the purification of nucleic acids. The device comprises a microfluidic channel (2) defined by a first surface (3) and a second surface (4) opposite the first surface, with connecting surfaces (5) joining the first and second surfaces. The cross-section through the microfluidic channel (2) is depicted as a rectangular space, with the fixed structures (6) uniformly distributed within this space. The connecting surfaces (5) are shown as the vertical boundaries of the channel (2), joining the first and second surfaces (3, 4) to form a closed channel through which the fluid flows along the z axis. Within the channel (2), a plurality of fixed structures (6) is attached to at least one of the first surface (3) or second surface (4). These fixed structures (6) are depicted as extending across at least 95% of the distance between the surface to which they are attached and the opposite surface. They are also depicted ensuring that they collectively occupy at least 95% of the distance between the connecting surfaces (5). The fixed structures (6) are shown as vertical pillars, which are integral to the device's function of capturing nucleic acids. The pillars are arranged in a staggered configuration, maximizing the interaction between the nucleic acids and the binding surfaces of the pillars.

The microfluidic device (1) includes at least one inlet (7) for introducing a fluid containing nucleic acids into the channel (2) and at least one outlet (8) for collecting the purified nucleic acids. The surfaces (9) of the fixed structures (6) are capable of reversibly binding nucleic acids or molecular assemblies comprising nucleic acids, which is advantageous for the purification process. The relative positions and sizes of the elements are such that the fixed structures (6) occupy a significant portion of the channel's height, ensuring that the nucleic acids have ample opportunity to interact with the binding surfaces (9) as they flow through the device. The first surface (3) and second surface (4) are parallel to each other, and the fixed structures (6) are perpendicular to these surfaces, extending vertically within the channel (2).

Referring to Figure 2, it illustrates a vertical cross-sectional view of a microfluidic device (1) similar to the one depicted in Figure 1. It is also designed for the purification of nucleic acids. The device comprises a microfluidic channel (2) defined by a first surface (3) and a second surface (4) opposite the first surface, with connecting surfaces (5) joining the first and second surfaces. Within this channel (2), a plurality of fixed structures (6) is depicted, extending vertically and occupying the entire distance between the first surface (3) and the second surface (4). These fixed structures (6) are represented as vertical pillars, uniformly spaced and extending across the entire height of the channel (2). The vertical extent of the pillars ensures that the entire height of the channel (2) is utilized, preventing any preferential flow paths that could bypass the purification structures. Also, they are depicted ensuring that they collectively occupy at least 95% of the distance between the connecting surfaces (5).

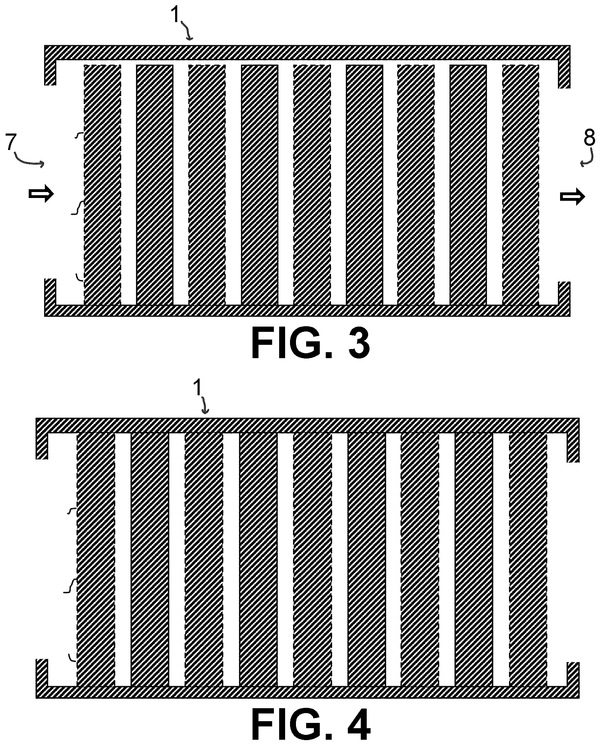

Referring to Figure 3, it illustrates a vertical longitudinal cross-sectional view of the microfluidic device (1) of Figure 1. The channel (2) is shown equipped with at least one inlet (7) for introducing a fluid containing nucleic acids or molecular assemblies comprising nucleic acids, and at least one outlet (8) for collecting the purified nucleic acids.

Referring to Figure 4, it illustrates a vertical longitudinal cross-sectional view of the microfluidic device (1) of Figure 2. The channel (2) is shown equipped with at least one inlet (7) for introducing a fluid containing nucleic acids or molecular assemblies comprising nucleic acids, and at least one outlet (8) for collecting the purified nucleic acids.

In embodiments, the surface (9) capable of reversibly binding nucleic acids or molecular assemblies comprising nucleic acids may comprise trityl groups. Trityl is a broader term that encompasses DMT and other similar protecting groups used in nucleic acid synthesis. In embodiments, the density and/or arrangement of said fixed structures (6) may be such that, when the binding surfaces (9) are free of bound nucleic acids, passing 1 μL of a sample containing 1 ng/μL of nucleic acids or molecular assemblies comprising nucleic acids through the microfluidic channel (2) at a flow rate of 1 μL/min results in the capture of at least 50% of the nucleic acids or molecular assemblies by the fixed structures (6). This allows for efficient capture of nucleic acids.

In embodiments, the fixed structures (6) may be three-dimensional structures forming an array. This provides a high surface area for binding. In embodiments, the average distance between a fixed structure (6) and its closest neighbor may be from 1 μm to 200 μm, preferably from 5 μm to 100 μm, yet more preferably from 5 μm to 50 μm. This spacing optimizes capture efficiency.

In embodiments, the fixed structures (6) may be pillars. Pillars are easy to fabricate.

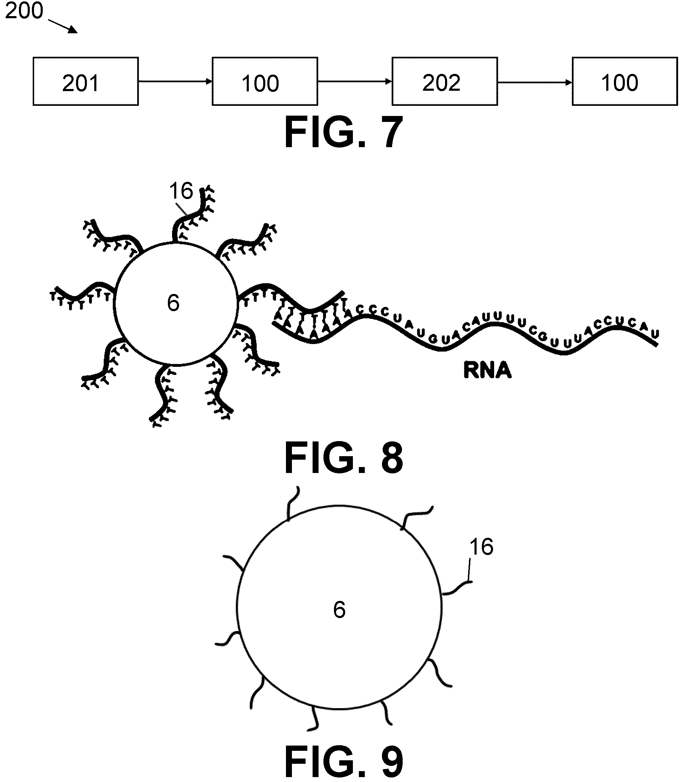

Referring to Figure 8, it illustrates a top view of a fixed structure (6) within a microfluidic device (1) designed for the purification of nucleic acids, according to a specific embodiment. The fixed structure (6) is depicted as a circular pillar, centrally positioned within the drawing. The surface (9) of the pillar is functionalized with nucleic acid sequences (16) that are complementary to the target nucleic acids intended for purification. These nucleic acid sequences (16) are represented as small, irregular protrusions extending outward from the circumference of the pillar. The reference number (6) is centrally located within the pillar, while the reference number (16) is placed adjacent to one of the protrusions, identifying the functionalized nucleic acid sequences. The drawing emphasizes the interaction between the pillar's surface (9) and the target nucleic acids, showcasing the pillar's role in capturing and binding the nucleic acids through complementary base pairing.

Referring to Figure 9, it illustrates a top view of a pillar (6) functionalized with C18 chains (16), which are hydrophobic groups designed to capture nucleic acids or molecular assemblies comprising nucleic acids.

In embodiments, the fixed structures (6) may comprise a porous material with pores having a pore size of from 50 to 500 nm. The pore size can be tuned for different nucleic acid lengths. In embodiments, the porous material may have a pore size of from 50 to 60 nm, and the device may be for purification of nucleic acids less than 35 nucleotides in length. This pore size is optimal for short nucleic acids. In embodiments, the porous material may have a pore size of from 61 nm to 199 nm, and the device may be for purification of nucleic acids greater than 35 nucleotides and up to 80 nucleotides in length. This pore size range suits medium length nucleic acids. In embodiments, the porous material may have a pore size of from 200 to 300 nm, and the device may be for purification of nucleic acids greater than 80 nucleotides in length. Larger pores accommodate longer nucleic acids.

In embodiments, the fixed structures (6) may comprise a mesh. A mesh structure provides a high surface area. In embodiments, the fixed structures (6) may be attached to both the first surface (3) and the second surface (4). This creates a dense array of structures. In embodiments, the fixed structures (6) may be arranged in a regular configuration, preferably in a regular staggered configuration. A staggered arrangement promotes interaction between the fluid and structures.

In embodiments, the fixed structures (6) may be made of the same material as a substrate (13) comprising the first surface (3) of the microfluidic device. Using the same material simplifies fabrication. In embodiments, the fixed structures (6) may be made of silicon or metal, preferably silicon. Silicon is readily micromachined. In embodiments, the surface (9) capable of reversibly binding either nucleic acids or molecular assemblies comprising nucleic acids may comprise entities (16) selected from the list consisting of hydrophobic groups such as C18 chains, nucleic acid sequences complementary to the target nucleic acids to be purified, streptavidin, or biotin. These have the advantage that they can be reused.

In embodiments, the microfluidic device (1) may comprise at least two outlets (8). Multiple outlets allow collection of different fractions. In embodiments, at least one of the outlets (8) may be fluidly connected to at least one of the inlets (7) to recirculate fluid through the channel (2). Recirculation can improve capture efficiency. In embodiments, the microfluidic device (1) may comprise at least two inlets (7). Multiple inlets enable introduction of different fluids.

Any feature of any embodiment of the first aspect may be as correspondingly described in any embodiment of any of the other aspects.

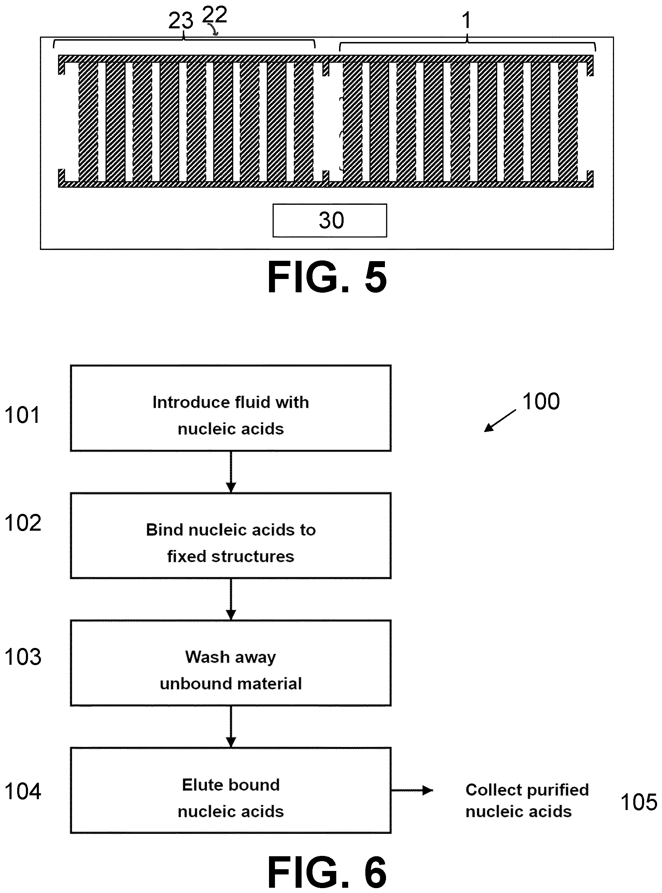

In the second aspect, the present invention relates to a method (100) of purifying nucleic acids comprising: introducing (101) a fluid containing nucleic acids or molecular assemblies comprising nucleic acids into a microfluidic device (1) according to any embodiments of the first aspect; allowing (102) the nucleic acids to bind to the fixed structures (6); washing away (103) unbound material; and eluting (104) the bound nucleic acids to collect (105) purified nucleic acids. This is illustrated in Figure 6. Referring to Figure 6, it illustrates a flowchart of a method (100) of purifying nucleic acids according to embodiments of the present invention. The flowchart is composed of a series of rectangular blocks connected by arrows, indicating the sequence of steps involved in the purification process. The first block, labeled "101," represents the step of introducing a fluid containing nucleic acids or molecular assemblies comprising nucleic acids into a microfluidic device (1). This step is followed by the block labeled "102," which denotes allowing the nucleic acids to bind to the fixed structures (6) within the microfluidic channel (2). The next block, labeled "103," signifies the washing away of unbound material, ensuring that only the desired nucleic acids remain bound to the fixed structures (6). Subsequently, the block labeled "104" represents the elution of the bound nucleic acids, which involves introducing a cleavage reagent or providing heat to release the nucleic acids from the fixed structures (6). Finally, the block labeled "105" indicates the collection of the purified nucleic acids, completing the purification process. The arrows between the blocks depict the flow of the process.

In embodiments, the washing step (103) may include introducing a wash buffer through the channel (2). The wash buffer removes unbound material. In embodiments, the microfluidic device (1) may comprise at least two inlets (7), wherein the introducing step (101) may be performed into a first inlet (7) and the washing step (103) may include introducing the wash buffer through the channel (2) via a second inlet (7), different from the first inlet (7). Using separate inlets prevents mixing of the sample and wash buffer. In embodiments, the eluting step (104) may include introducing a cleavage reagent through the channel (2), providing heat to the bound nucleic acids, or both. The cleavage reagent or heat releases the bound nucleic acids. In embodiments, the fluid containing nucleic acids may be introduced into the channel (2) under pressure-driven flow. Pressure-driven flow enables control over the flow rate.

Any feature of any embodiment of the second aspect may be as correspondingly described in any embodiment of any of the other aspects.

In the third aspect, the present invention relates to a process (200) for nucleic acid synthesis, comprising: synthesizing (201) nucleic acid fragments; and purifying (100) the synthesized nucleic acid fragments using the method (100) according to any embodiments of the second aspect. Referring to Figure 7, it illustrates a schematic view of the process (200) for nucleic acid synthesis according to embodiments of the present invention. The drawing depicts

- Synthesizing (201) nucleic acid fragments using known chemical or enzymatic methods.
- Purifying (100) the synthesized fragments using the microfluidic device (1), where the surfaces (9) are functionalized with entities (16) that facilitate binding.

In embodiments, the surface (9) may comprise entities (16) selected from the list consisting of hydrophobic groups such as C18 chains, streptavidin, and biotin. Preferably hydrophobic groups when the synthesized fragments comprise trityl groups at the 5'-end of oligonucleotides. In embodiments, the process (200) may further comprise the following steps after the purifying step (100): assembling (202) the purified nucleic acid fragments into larger nucleic acid fragments, then purifying (100) the assembled larger nucleic acid fragments using the method (100) according to any embodiments of the second aspect, wherein the surface (9) capable of reversibly binding either nucleic acids or molecular assemblies comprising nucleic acids comprises nucleic acid sequences complementary to the target nucleic acids to be purified, wherein completely assembled fragments are retained and incompletely assembled fragments are removed. This allows purification of the assembled products.

Any feature of any embodiment of the third aspect may be as correspondingly described in any embodiment of any of the other aspects.

In the fourth aspect, the present invention relates to a system (22) for synthesis and purification of nucleic acids comprising: a synthesis module (23) for synthesizing nucleic acids fragments; and a first microfluidic device (1) according to any embodiments of the first aspect; wherein the synthesis module (23) and microfluidic device (1) are fluidically connected.

Referring to Figure 5, it illustrates a schematic view of a system (22) for the synthesis and purification of nucleic acids. The system (22) comprises a synthesis module (23) and a first microfluidic device (1) fluidically connected in series. The synthesis module (23) is depicted on the left side of the drawing, indicating the initial stage of the process where nucleic acid fragments are synthesized. This module (23) is connected via a conduit to the first microfluidic device (1), which is represented on the right side of the drawing, indicating the subsequent purification stage.

Within the first microfluidic device (1), there is a depiction of a microfluidic channel (2) that runs horizontally. The channel (2) has a first surface (3) and a second surface (4) opposite the first surface, with connecting surfaces (5) joining the first and second surfaces. Inside the channel (2), a plurality of fixed structures (6) is shown, which are attached to at least one of the first surface (3) or second surface (4) of the channel. These fixed structures (6) are illustrated as vertical rectangles extending across the channel (2).

Additionally, the system (22) comprises a control unit (30) for the automated operation of the synthesis and purification processes. The control unit (30) is represented as a smaller rectangular box. In embodiments, the system (22) may comprise multiple microfluidic purification devices (1) connected in series to achieve higher purification rates. Connecting devices (1) in series allows for multi-stage purification. An example of multi-stage purification is where, first, one end of the nucleic acid molecule, such as the 3'-end, is captured, and all molecules that lack this end are washed away. After the release of the captured products, their 5'-end is then captured. Again, all molecules that don't have this end, e.g., due to synthesis or fragment assembly failures, are washed away. This multi-stage purification in which both ends of the target molecule are captured in series allows to confirm their completion. There is also the possibility of including an assembling module (202) for assembling the purified nucleic acid fragments into larger nucleic acid fragments, and a second microfluidic device (1) for further purification. However, these additional components are not explicitly depicted in Figure 5. They are depicted in Figure 7.

In embodiments, the surface (9) capable of reversibly binding either nucleic acids or molecular assemblies comprising nucleic acids may comprise entities (16) selected from the list consisting of hydrophobic groups such as C18 chains, streptavidin, and biotin. These entities are suitable for purifying synthesized fragments. In embodiments, the system (22) may further comprise an assembling module (202) for assembling the purified nucleic acid fragments into larger nucleic acid fragments, and a second microfluidic device (1) according to any embodiments of the first aspect, wherein the surface (9) capable of reversibly binding either nucleic acids or molecular assemblies comprising nucleic acids comprises nucleic acid sequences complementary to the target nucleic acids to be purified. This enables a complete workflow from synthesis to assembly and purification.

In embodiments, the synthesis module may comprise a channel having a first surface, a second surface opposite the first surface, and connecting surfaces joining the first and second surfaces; an ensemble of one or more fixed structures within said channel, each structure being attached to at least one of the first surface or second surface of the channel, wherein the ensemble, including any spaces between individual structures, collectively extends over at least 95% of the distance between the connecting surfaces of the channel, and each structure extending across at least 95% of the distance between the surface to which it is attached and the opposite surface. The fixed structures in the synthesis module provide a high surface area for efficient synthesis. In embodiments, each fixed structure may be structurally identical to the fixed structures within the microfluidic channel of the microfluidic deice for purification of nucleic acids.

In embodiments, the system (22) may further comprise a control unit (30) for automated operation of the synthesis and purification processes. Automation improves efficiency and reproducibility. In embodiments, the synthesis module (23) may synthesize nucleic acids using one of: phosphoramidite chemistry or enzymatic synthesis controlled through reagent, light, heat, and/or electrochemistry. These are common nucleic acid synthesis methods.

Any feature of any embodiment of the fourth aspect may be as correspondingly described in any embodiment of any of the other aspects.

In the fifth aspect, the present invention relates to a computer program comprising instructions to cause the system (22) according to embodiments of the fourth aspect to execute the steps of the process (200) according to embodiments of the third aspect.

In the sixth aspect, the present invention relates to a computer-readable medium having stored thereon the computer program according to the fifth aspect.

Example 1: Purification of Nucleic Acids Using Pillared Microfluidic Devices

This example demonstrates the use of pillared microfluidic devices for the purification of nucleic acids, specifically DNA, RNA and XNA strands. The significance of this experiment lies in the potential to transpose the processes currently performed on beads to a microfluidic device, allowing for on-line purification using pillars, thereby reducing sample manipulation and hands-on time.

The materials used in this experiment include a microfluidic device with at least one microfluidic channel, a plurality of fixed pillar structures within the channel, at least one inlet for introducing a fluid containing nucleic acids, and at least one outlet for collecting purified nucleic acids. The pillar structures are functionalized with either C18 chains or nucleic acid sequences complementary to the target nucleic acids to be purified.

The experimental setup involves introducing a fluid containing nucleic acids into the microfluidic device through the inlet under pressure-driven flow. The nucleic acids are allowed to bind to the functionalized pillar structures. Unbound material is then washed away by introducing a wash buffer through the channel. Finally, the bound nucleic acids are eluted by introducing a cleavage reagent or providing heat to collect the purified nucleic acids. Figure 8 illustrates how RNA molecules can be captured by the pillars functionalized with complementary nucleic acid sequences, while Figure 9 shows pillars functionalized with C18 chains able to retain DNA strands with 5'-O-DMT protecting groups. 1 $cm^2$ of the pillared chip can capture $10^{12}$ molecules.

Example 2: Purification of Short Nucleic Acid Fragments Using Porous Fixed Structures

In this example, a microfluidic device with fixed pillars made of a porous material is used to purify short nucleic acid fragments. The porous material has a pore size of 55 nm, making it suitable for purification of nucleic acids less than 35 nucleotides in length.

The microfluidic device is prepared with a channel containing fixed pillars made of a porous silicon material. The porous structures are functionalized with C18 chains to enable reversible binding to the 5'-O-DMT protecting groups of the short nucleic acid fragments. A sample containing a mixture of short nucleic acid fragments (20-40 nucleotides) is introduced into the device under pressure-driven flow at a rate of 1 $\mu$L/min.

As the sample flows through the device, the short nucleic acid fragments interact with the C18-functionalized porous structures. The fragments less than 35 nucleotides in length are captured within the pores, while larger fragments pass through. After the sample introduction, a wash buffer is flowed through the device to remove any unbound material.

To elute the captured nucleic acids, a cleavage reagent is introduced into the channel. The purified short nucleic acid fragments are then collected from the outlet.

Example 3: Automated Nucleic Acid Synthesis and Purification System

This example illustrates the use of an integrated system for nucleic acid synthesis and purification, combining a synthesis and assembly modules with two purification modules based on pillared microfluidic devices.

The system consists of:

1. A synthesis module for chemical synthesis of nucleic acid fragments
2. A first purification module with C18-functionalized pillars
3. An assembly module for joining purified fragments
4. A second purification module with pillars functionalized with complementary nucleic acid sequences
5. A control unit for automated operation

The synthesis module chemically synthesizes short nucleic acid fragments. These fragments are then automatically transferred to the first purification module, where the C18-functionalized pillars capture the synthesized fragments. After washing and elution, the purified fragments are moved to the assembly module.

In the assembly module, the purified short fragments are joined to create larger nucleic acid constructs. These assembled constructs are then transferred to the second purification module. Here, pillars functionalized with complementary sequences first capture the 3'-end of the nucleic acid molecules, allowing incomplete molecules lacking this region to be washed away. After release of the captured products, a second capture step targets the 5'-end, enabling removal of any molecules that lack this end due to synthesis or assembly failures. This multi-stage purification approach confirms the completion of the target molecules.

The entire process is controlled by the automated control unit, which manages the timing of each step, fluid flow rates, and temperature conditions where necessary.

Example 4: Purification of Large Nucleic Acids Using Wide-Pore Fixed Structures

This experiment focuses on the purification of large nucleic acid molecules using a microfluidic device with a single fixed mesh structure. The mesh structure in this case has a pore size of 250 nm, suitable for purification of nucleic acids greater than 80 nucleotides in length.

The microfluidic device is prepared with a channel containing the mesh attached to an inner wall of the channel. It is made of a porous polymer material. The surfaces of the mesh are functionalized with streptavidin. A sample containing a mixture of nucleic acid fragments, including large fragments over 100 nucleotides in length, is prepared. These large fragments are biotinylated at one end.

The sample is introduced into the device at a flow rate of 0.5 μL/min. As the sample flows through, the biotinylated large nucleic acid fragments bind to the streptavidin-functionalized porous structures. Smaller fragments and other impurities pass through the device. After sample introduction, a wash buffer is used to remove any unbound material.

To elute the captured large nucleic acids, a biotin-containing buffer is flowed through the device, competing with the immobilized nucleic acids for binding to streptavidin. The eluted, purified large nucleic acid fragments are collected from the outlet.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A microfluidic device (1) for purification of nucleic acids comprising:

   at least one microfluidic channel (2), said channel having a first surface (3), a second surface (4) opposite the first surface, and connecting surfaces (5) joining the first and second surfaces;
   a plurality of fixed structures (6) within said channel, said structures being attached to at least one of the first surface or second surface of the channel, collectively occupying at least 95% of the distance between the connecting surfaces, and each structure extending across at least 95% of the distance between the surface to which it is attached and the opposite surface;
   at least one inlet (7) for introducing a fluid containing nucleic acids or molecular assemblies comprising nucleic acids into the channel; and
   at least one outlet (8) for collecting purified nucleic acids;
   wherein said fixed structures comprise a surface (9) capable of reversibly binding either nucleic acids or molecular assemblies comprising nucleic acids.

2. The microfluidic device (1) according to claim 1, wherein the fixed structures (6) are pillars (6).

3. The microfluidic device (1) according to any one of the preceding claims, wherein the fixed structures (6) are attached to both the first surface (3) and the second surface (4).

4. The microfluidic device (1) according to any one of the preceding claims, wherein the fixed structures (6) are arranged in a regular configuration, preferably in a regular staggered configuration.

5. The microfluidic device (1) according to any one of the preceding claims, wherein the fixed structures (6) are made of the same material as a substrate (13) comprising the first surface (3) of the microfluidic device.

6. The microfluidic device (1) according to any one of the preceding claims, wherein the fixed structures (6) are made of silicon or metal, preferably silicon.

7. The microfluidic device (1) according to any one of the preceding claims, wherein the surface (9) capable of reversibly binding either nucleic acids or molecular assemblies comprising nucleic acids comprises entities (16) selected from the list consisting of hydrophobic groups such as C18 chains, nucleic acid sequences complementary to the target nucleic acids to be purified, streptavidin, and biotin.

8. A method (100) of purifying nucleic acids comprising:

   Introducing (101) a fluid containing nucleic acids or molecular assemblies comprising nucleic acids into a microfluidic device (1) according to any one of claims 1 to 7;
   Allowing (102) the nucleic acids to bind to the fixed structures (6);
   washing away (103) unbound material; and
   eluting (104) the bound nucleic acids to collect (105) purified nucleic acids.

9. The method according to claim 8, wherein the washing step (103) includes introducing a wash buffer through the channel (2).

10. The method according to claim 8 or 9, wherein the eluting step includes introducing a cleavage reagent through the channel (2), providing heat to the bound nucleic acids, or both.

11. The method according to any one of claims 8 to 10, wherein the fluid containing nucleic acids is introduced into the channel (2) under pressure-driven flow.

**12.** A process (200) for nucleic acid synthesis and assembly, comprising:

> Synthesizing (201) nucleic acid fragments; and Purifying (100) the synthesized nucleic acid fragments using the method of any one of claims 8 to 11.

**13.** A system (22) for synthesis and purification of nucleic acids comprising:

> a synthesis module (23) for synthesizing nucleic acids fragments; and
> a first microfluidic device (1) according to any one of claims 1 to 7;
> wherein the synthesis module and microfluidic device (1) are fluidically connected.

**14.** The system (22) according to claim 13, further comprising an assembling module for assembling the purified nucleic acid fragments into larger nucleic acid fragments, and a second microfluidic device (1) according to any one of claims 1 to 7 wherein the surface (9) capable of reversibly binding either nucleic acids or molecular assemblies comprising nucleic acids comprises nucleic acid sequences complementary to the target nucleic acids to be purified.

**15.** The system (22) according to any one of claims 13 to 14, further comprising a control unit (30) for automated operation of the synthesis and purification processes.

# FIG. 1

# FIG. 2

**FIG. 3**

**FIG. 4**

23 22                                    1

30

# FIG. 5

101 | Introduce fluid with
nucleic acids

100

102 | Bind nucleic acids to
fixed structures

103 | Wash away
unbound material

104 | Elute bound
nucleic acids     →    Collect purified
nucleic acids   105

# FIG. 6

200

| 201 | → | 100 | → | 202 | → | 100 |

**FIG. 7**

16

6

CCCU A U G U A CAUUUUCG U U U CCUCAU

RNA

**FIG. 8**

16

6

**FIG. 9**

Europäisches Patentamt
European Patent Office
Office européen des brevets

# EUROPEAN SEARCH REPORT

**Application Number**

EP 24 20 9261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/309346 A1 (STAKENBORG TIM [BE] ET AL) 10 October 2019 (2019-10-10) * paragraphs [0081] - [0090], [0102]; figures 1,16-19 * | 1-6 | INV. B01L3/00 C12N15/10 C12Q1/68 G01N1/40 |
| X | US 2006/068491 A1 (MAKINO YOSHIHIKO [JP] ET AL) 30 March 2006 (2006-03-30) * paragraphs [0002], [0106] - [0109], [0208], [0209]; figures 5A, 5B; example 4 * | 1-6 | |
| X | US 2015/321191 A1 (KENDALL ERIC L [US] ET AL) 12 November 2015 (2015-11-12) * paragraphs [0015] - [0017], [0094] - [0102] * * paragraphs [0133] - [0135]; figures 9,23-26 * | 1,3-6 | |
| X | US 2023/303996 A1 (SASSER JACOB SHANE [US] ET AL) 28 September 2023 (2023-09-28) * paragraphs [0029] - [0038]; figures 1-10 * * paragraphs [0054], [0063] * | 1,7 | TECHNICAL FIELDS SEARCHED (IPC) B01L C40B G01N C12Q C12N |
| A | US 2003/224436 A1 (NELSON ROBERT J [US] ET AL) 4 December 2003 (2003-12-04) * paragraphs [0046], [0050] - [0052] * | 7 | |
| A | PAMELA N. NGE ET AL: "Advances in Microfluidic Materials, Functions, Integration, and Applications", CHEMICAL REVIEWS, vol. 113, no. 4, 10 April 2013 (2013-04-10), pages 2550-2583, XP055102961, ISSN: 0009-2665, DOI: 10.1021/cr300337x * page 2559 - page 2561 * * page 2572 * | 7 | |

The present search report has been drawn up for all claims

| Place of search | Date of completion of the search | Examiner |
|---|---|---|
| The Hague | 18 July 2025 | Tiede, Ralph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                             

& : member of the same patent family, corresponding document

EPO FORM 1503 03.82 (P04C01)

2

Europäisches
Patentamt

European
Patent Office

Office européen
des brevets

**Application Number**

EP 24 20 9261

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

    see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

    2-7(completely); 1(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

**Application Number**

EP 24 20 9261

The Search Division considers that the present European patent application does not comply with the requirements of unity of invention and relates to several inventions or groups of inventions, namely:

1. claims: 2-6(completely); 1(partially)

A microfluidic device (1) for purification of nucleic acids comprising: at least one microfluidic channel (2), said channel having a first surface (3), a second surface (4) opposite the first surface, and connecting surfaces (5) joining the first and second surfaces; a plurality of fixed structures (6) within said channel, said structures being attached to at least one of the first surface or second surface of the channel, collectively occupying at least 95% of the distance between the connecting surfaces, and each structure extending across at least 95% of the distance between the surface to which it is attached and the opposite surface; at least one inlet (7) for introducing a fluid containing nucleic acids or molecular assemblies comprising nucleic acids into the channel; and at least one outlet (8) for collecting purified nucleic acids; wherein said fixed structures comprise a surface (9) capable of reversibly binding either nucleic acids or molecular assemblies comprising nucleic acids. Further comprising details about physical structures between first and second surfaces, in order to provide alternative structures to reduce diffusion times.

---

2. claims: 7(completely); 1(partially)

A microfluidic device (1) for purification of nucleic acids comprising: at least one microfluidic channel (2), said channel having a first surface (3), a second surface (4) opposite the first surface, and connecting surfaces (5) joining the first and second surfaces; a plurality of fixed structures (6) within said channel, said structures being attached to at least one of the first surface or second surface of the channel, collectively occupying at least 95% of the distance between the connecting surfaces, and each structure extending across at least 95% of the distance between the surface to which it is attached and the opposite surface; at least one inlet (7) for introducing a fluid containing nucleic acids or molecular assemblies comprising nucleic acids into the channel; and at least one outlet (8) for collecting purified nucleic acids; wherein said fixed structures comprise a surface (9) capable of reversibly binding either nucleic acids or molecular assemblies comprising nucleic acids. Further comprising details about chemical nature of the binding surfaces, in order to improve nucleic acid binding for purification.

---

3. claims: 9-11(completely); 8(partially)

page 1 of 2

Europäisches
Patentamt

European
Patent Office

Office européen
des brevets

**LACK OF UNITY OF INVENTION
SHEET B**

**Application Number**

**EP 24 20 9261**

The Search Division considers that the present European patent application does not comply with the requirements of unity of invention and relates to several inventions or groups of inventions, namely:

A method (100) of purifying nucleic acids comprising: Introducing (101) a fluid containing nucleic acids or molecular assemblies comprising nucleic acids into a microfluidic device (1) according to any one of claims 1 to 7;Allowing (102) the nucleic acids to bind to the fixed structures (6); washing away (103) unbound material; and eluting (104) the bound nucleic acids to collect (105) purified nucleic acids. Further comprising details about purification method steps and operating conditions, in order to provide alternative purification methods for nucleic acids.

---

4. claims: 12-15(completely); 1, 8(partially)

A process (200) for nucleic acid synthesis and assembly, comprising: Synthesizing (201) nucleic acid fragments; and Purifying (100) the synthesized nucleic acid fragments using the method of any one of claims 8 to 11. Addressing the problem to synthesize nucleic acid fragments.

---

## ANNEX TO THE EUROPEAN SEARCH REPORT
## ON EUROPEAN PATENT APPLICATION NO.

EP 24 20 9261

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2025

| Patent document cited in search report | Publication date | Patent family member(s) | | Publication date |
|---|---|---|---|---|
| US 2019309346 A1 | 10-10-2019 | EP | 3338889 A1 | 27-06-2018 |
| | | EP | 3558525 A1 | 30-10-2019 |
| | | US | 2019309346 A1 | 10-10-2019 |
| | | WO | 2018115040 A1 | 28-06-2018 |
| US 2006068491 A1 | 30-03-2006 | JP | 2006087372 A | 06-04-2006 |
| | | US | 2006068491 A1 | 30-03-2006 |
| US 2015321191 A1 | 12-11-2015 | US | 2015321191 A1 | 12-11-2015 |
| | | US | 2020353460 A1 | 12-11-2020 |
| | | WO | 2015195178 A2 | 23-12-2015 |
| US 2023303996 A1 | 28-09-2023 | US | 2023303996 A1 | 28-09-2023 |
| | | WO | 2022046079 A1 | 03-03-2022 |
| US 2003224436 A1 | 04-12-2003 | AU | 758140 B2 | 13-03-2003 |
| | | CA | 2320362 A1 | 12-08-1999 |
| | | EP | 1053298 A1 | 22-11-2000 |
| | | JP | 2002502597 A | 29-01-2002 |
| | | US | 6074827 A | 13-06-2000 |
| | | US | 6344326 B1 | 05-02-2002 |
| | | US | 2002119482 A1 | 29-08-2002 |
| | | US | 2003224436 A1 | 04-12-2003 |
| | | WO | 9940174 A1 | 12-08-1999 |

For more details about this annex : see Official Journal of the European Patent Office, No. 12/82